# FASCICULE DE BREVET EUROPEEN

(11) **EP 0 465 359 B1**
(45) Date de publication et mention de la délivrance du brevet: **29.03.1995**
(21) Numéro de dépôt: 91401834.6
(22) Date de dépôt: 03.07.1991
(51) Int. Cl.: A61B 6/00

(54) **Système de radiodiagnostic pour examen angiographique avec dispositif automatique de suivi d'embole**
Röntgenstrahl-diagnostisches Verfahren zur angiographischen Untersuchung mit Einrichtung zur automatischen Verfolgung der Embolie
X-ray diagnostic system for angiographic examination with an automatic device for a subsequent embolism

(30) Priorité: 06.07.1990 FR 9008624
(43) Date de publication de la demande: 08.01.1992
(73) Titulaire: GENERAL ELECTRIC CGR S.A., F-92130 Issy les Moulineaux (FR)
(72) Inventeur: KLausz, Rémy, F-75116 Paris (FR)
(74) Mandataire: Ballot, Paul Denis Jacques

(56) Documents cités:
- EP-A- 0 146 991
- EP-A- 0 374 328
- DE-A- 3 203 594
- DE-B- 1 125 560

## Description

L'invention concerne les systèmes de radiodiagnostic et, plus particulièrement, ceux qui sont utilisés pour les examens angiographiques des membres inférieurs. Elle concerne également, dans lesdits systèmes, un dispositif qui permet de suivre le déplacement du produit de contraste ou embole et réaliser les clichés radiologiques en synchronisme avec ledit déplacement.

L'angiographie est la technique de radiodiagnostic appliquée et adaptée au réseau vasculaire : artères, veines, tissus perfusés. Elle fait appel à des liquides à base d'iode, dits de contraste, opaques aux rayons X, et injectés dans le réseau vasculaire afin de permettre sa visualisation par différenciation avec les tissus environnants. De manière plus précise, le patient est allongé sur une table qui est, par exemple, prévue de manière à se déplacer sous une source de rayonnement X associée à un récepteur disposé de l'autre côté du patient par rapport à la source. Dans d'autres systèmes de radiodiagnostic, la table est fixe tandis que l'ensemble source de rayonnement-récepteur est mobile.Le praticien injecte une dose de produit de contraste dans une artère ou une veine du patient allongé sur la table. Puis quelques secondes après cette injection, il réalise plusieurs clichés radiographiques successifs du patient de manière à visualiser les vaisseaux sanguins et mesurer la progression au cours du temps de la dose de produit de contraste, appelée embole, dans lesdits vaisseaux sanguins.

Lorsque l'examen angiographique est limité à une partie du corps qui correspond au champ du récepteur, c'est-à-dire aux dimensions du film radiographique ou de l'intensificateur d'images radiologiques, les clichés sont effectués sans déplacer le patient, c'est-à-dire la table, par rapport à l'ensemble source-récepteur ou vice-versa.

Lorsque l'examen angiographique concerne les membres inférieurs, c'est-à-dire une longueur de 120 centimètres environ, trois procédés sont actuellement mis en oeuvre.

Un premier procédé consiste à utiliser un film radiographique de grande longueur de manière à couvrir la totalité des membres inférieurs en un seul cliché et à réaliser plusieurs clichés successifs, par exemple six, séparés par des intervalles de temps qui permettent d'enregistrer l'image de la progression de l'embole. Pour chaque cliché, on irradie toute la surface des membres inférieurs, ce qui conduit à une exposition importante aux rayons X préjudiciable au patient et à un coût relativement élevé qui est dû au prix des films radiographiques de grand format. En outre, les dispositifs qui sont généralement utilisés disposent d'un nombre de clichés limité, six en général, ce qui oblige à cadencer la prise des clichés en prévoyant au mieux le passage de l'embole. Enfin, la zone irradiée du patient est inutilement grande, plusieurs images étant réalisées avant ou après l'arrivée du produit de contraste en chaque point. Ceci oblige à allonger la durée de passage du produit de contraste et donc, pour conserver la même concentration, à augmenter le volume injecté, ce qui peut être nuisible au patient par suite de la toxicité du produit et augmente le coût de l'examen.

Un deuxième procédé consiste à examiner les membres inférieurs zone par zone, chaque zone correspondant par exemple à un champ utile de 35 x 35 cm ou à un diamètre de 30 ou 35 cm. Pour cela, on effectue une première injection du produit de contraste et on réalise plusieurs clichés successifs de la zone proche de l'injection. Ensuite, on déplace la table ou l'ensemble source-récepteur pour centrer les images sur une zone voisine. On peut alors recommencer les mêmes opérations : injection du produit de contraste et enregistrement des images. On recommence ensuite le processus en totalité pour les zones suivantes. Ainsi, chaque série de clichés d'une zone permet de voir du début à la fin le passage de l'embole dans le réseau sanguin de ladite zone. Un tel procédé conduit à effectuer plusieurs injections (en pratique autant que de positions et de zones radiographiées), ce qui prend du temps, occasionne une gêne importante du patient et est onéreux en produit de contraste. Enfin, ce procédé conduit à réaliser un grand nombre d'expositions et il en résulte une irradiation supplémentaire du patient et une augmentation du coût de l'examen quand le récepteur est constitué par un film radiographique.

Un troisième procédé consiste à effectuer une seule injection du produit de contraste et à réaliser un déplacement relatif entre le patient et l'ensemble source-récepteur de manière que la zone du patient située en regard de l'ensemble source-récepteur soit constamment la zone où la concentration du produit de contraste dans le réseau sanguin permet d'en réaliser les images souhaitées. Ce troisième procédé exige une synchronisation optimale pour les déplacements entre les positions successives des clichés et le passage de l'embole. Actuellement, les tables de radiologie qui sont prévues pour ce genre d'examen ont une sélection d'avances suivant des pas fixes et à des intervalles de temps constants et dont la durée est à la discrétion du praticien. Elles ne permettent pas au praticien de facilement maîtriser les phases de l'examen de manière à suivre la progression de l'embole quels que soient le champ du récepteur, la morphologie du patient et les aspects pathologiques.

La description qui vient d'être faite des trois procédés d'examen angiographique montre que le problème de la détermination de la progression de l'embole le long du réseau sanguin des membres inférieurs est commune aux trois procédés même si les conséquences sont plus ou moins critiques selon le procédé considéré.

Pour suivre le passage de l'embole, il est connu que les praticiens utilisent les réactions et/ou sensations du patient au passage de l'embole, telles que la sensation de chaleur, la gêne, la douleur pour adapter en temps réel la prise des différents clichés à la progression de l'embole.

Un but de la présente invention est donc de réaliser un dispositif de détection de la progression de l'embole qui ne requiert pas la collaboration du patient, ni la même attention du praticien.

Un autre but de la présente invention est également de réaliser un système de radiodiagnostic pour examen angiographique qui comporte un tel dispositif de détection de la progression de l'embole de manière à synchroniser la prise des différents clichés avec la position de l'embole détectée par ledit dispositif.

L'invention est basée sur la constatation que le produit de contraste, par sa composition en sels organiques trisubstitués par des atomes d'iode, provoque des modifications physiologiques réversibles en relation avec la concentration en produit de contraste dans le sang à l'endroit concerné. De manière schématique, l'effet du produit de contraste peut se résumer, en ce qui concerne l'invention, par la notion de vaso-dilatation des vaisseaux sanguins de sorte qu'il en résulte une augmentation de la circulation sanguine périphérique par dilatation des petits vaisseaux. Ce phénomène se propage au fur et à mesure que progresse l'embole. C'est cette propriété qui sera mise à profit dans les dispositifs de détection de l'embole selon la présente invention.

L'invention concerne un système de radiodiagnostic pour examen angiographique d'un patient qui comprend un statif supportant une source de rayonnement X et un récepteur d'images radiologiques coopérant avec la source, une table de radiologie du type comportant un panneau monté sur un socle, des moyens pour obtenir des déplacements relatifs du statif et du panneau, un dispositif d'injection d'un produit de contraste dans le réseau sanguin et un dispositif d'acquisition des images détectées par le récepteur de manière à obtenir finalement une image radiologique du corps d'un patient, caractérisé en ce qu'il comprend en outre, des moyens pour détecter la progression du produit de contraste dans le réseau sanguin du patient et des moyens pour déclencher d'une part, le déplacement relatif du statif et du panneau en plusieurs positions et, d'autre part, la prise de l'image radiologique pour ces positions relatives de manière que l'image radiologique obtenue en chacune de ces positions relatives soit enregistrée à un instant où les vaisseaux sanguins de la zone dont on enregistre une image présentent une concentration significative en produit de contraste.

Les moyens pour détecter la progression du produit de contraste sont des capteurs qui sont sensibles aux effets physiologiques induits par le produit de contraste, tels que l'élévation de la température des tissus cutanés ou l'augmentation du volume sanguin des couches périphériques et donc de leur contenu en eau.

Dans le cas de l'élévation de température, les capteurs sont, par exemple, des thermocouples disposés sur le corps du patient, soit une caméra de thermographie disposée à distance.

Dans le cas d'une augmentation du volume d'eau à détecter, les capteurs peuvent être du type à résonance magnétique nucléaire, du type sensible aux variations de résistivité en courant continu ou alternatif basse fréquence ou encore aux variations d'impédance pour des champs électriques à très haute fréquence.

On peut aussi utiliser un produit de contraste radio-actif et détecter sa progression par un détecteur approprié tel qu'une série de détecteurs de rayonnement ionisant ou gamma caméra.

D'autres buts, caractéristiques et avantages de la présente invention apparaîtront à la lecture de la description suivante d'exemples particuliers de réalisation, ladite description étant faite en relation avec les dessins joints dans lesquels :
- la figure 1 est une vue schématique d'un système de radiodiagnostic pour examen angiographique selon l'art antérieur;
- la figure 2 est une vue schématique d'un système de radiodiagnostic, pour examen angiographique comportant un premier dispositif de suivi automatique d'embole selon la présente invention;
- les figures 3-a à 3-e sont des diagrammes montrant la forme des signaux détectés par les capteurs utilisés dans le premier dispositif de suivi automatique d'embole de la figure 2;
- la figure 4 est une vue schématique d'un système de radiodiagnostic, pour examen angiographique comportant un deuxième dispositif de suivi automatique d'embole selon la présente invention;
- la figure 5 est une vue schématique d'une variante du système de radiodiagnostic pour examen angiographique tel que décrit en relation avec la figure 4.

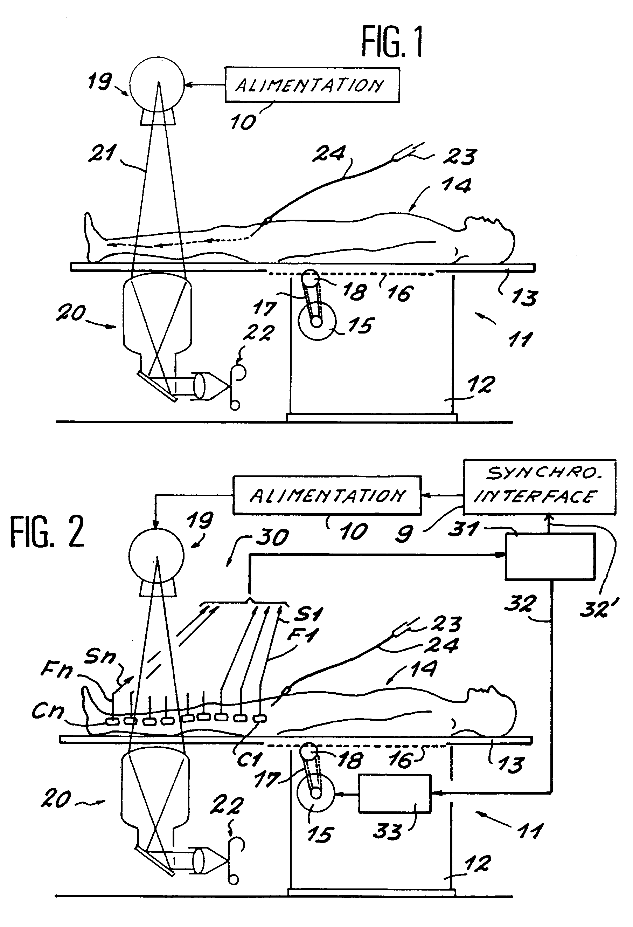

Sur la figure 1, un système de radiodiagnostic pour examen angiographique selon l'art antérieur comprend une table de radiologie 11 et un ensemble, appelé statif, comportant une source 19 de rayonnement X et un récepteur 20 d'images radiologiques. La source de rayonnement X est alimentée par un dispositif d'alimentation électrique 10 de type classique.

La table 11 comporte un socle 12 et un panneau 13 de support d'un patient 14. Le socle 12 comporte des moyens de déplacement longitudinal du panneau 13 matérialisés par un moteur 15 associé à une crémaillère 16 par l'intermédiaire d'une courroie 17 et d'une roue dentée 18.

L'ensemble source 19-récepteur 20 est porté par des moyens non représentés, qui peuvent consister en un arceau en forme de demi-arc de cercle par exemple dont une extrémité porte la source et dont l'autre extrémité porte le récepteur. L'arceau est lui-même porté par un bras articulé dont les mouvements permettent de déplacer l'ensemble source-récepteur par rapport à la table 11.

Dans la position représentée sur la figure 1, la source 19 émet un faisceau 21 de rayons X qui couvre les jambes du patient 14. Le rayonnement X, atténué par les jambes, est détecté par le récepteur 20, qui est par exemple ici, un amplificateur de luminance associé à une caméra photographique 22.

On comprend qu'en déplaçant le panneau 13 par rapport à l'ensemble source-récepteur, il est possible d'obtenir des clichés des différentes parties des membres inférieurs du patient.

Le produit de contraste est contenu dans une seringue 23 et est injecté dans l'artère fémorale du patient; dans le cas d'un examen angiographique des membres inférieurs, ce produit est injecté par l'intermédiaire d'un cathéter 24.

Au départ, le praticien positionne le panneau 13 pour amener l'ensemble source-récepteur au niveau de l'abdomen. Au moment de l'injection, il prend plusieurs clichés. Il le déplace ensuite vers les pieds d'une longueur déterminée par le champ du récepteur et prend à nouveau plusieurs clichés et ainsi de suite jusqu'à la position des pieds. Dans une telle manière de faire, c'est l'expérience du praticien qui lui permet de déterminer à quels instants il faut, d'une part, prendre les clichés et, d'autre part, déplacer le panneau 13 à la position suivante.

Dans la demande de brevet français n° 88 17523 déposée par la demanderesse le 30 décembre 1988 et intitulée : "SYSTEME DE RADIOLOGIE POUR EXAMEN ANGIOGRAPHIQUE ET PROCEDE DE MISE EN OEUVRE", l'examen angiographique proprement dit est précédé d'une phase de radioscopie de manière à déterminer et enregistrer différentes positions du panneau 13 en fonction de l'anatomie du patient. Au cours de l'examen réel, la table reprend les mêmes positions de manière automatique aux instants prédéterminés a priori par le praticien.

La présente invention propose de détecter le passage de l'embole de manière à réaliser automatiquement la prise des clichés et le déplacement relatif du patient et de l'ensemble source-récepteur pour suivre la progression de l'embole.

Pour détecter le passage de l'embole, l'invention propose d'utiliser des capteurs qui détectent certaines modifications physiologiques dues au produit de contraste, notamment la vaso-dilatation des vaisseaux sanguins.

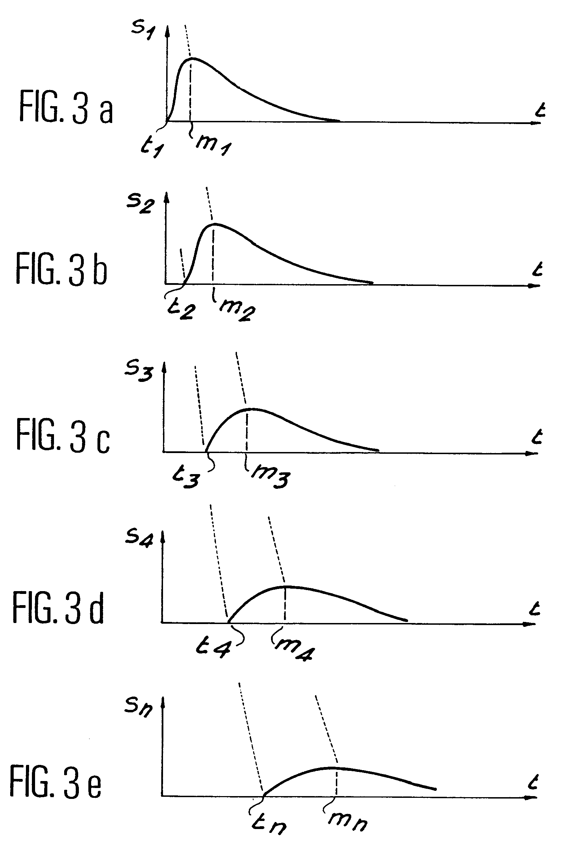

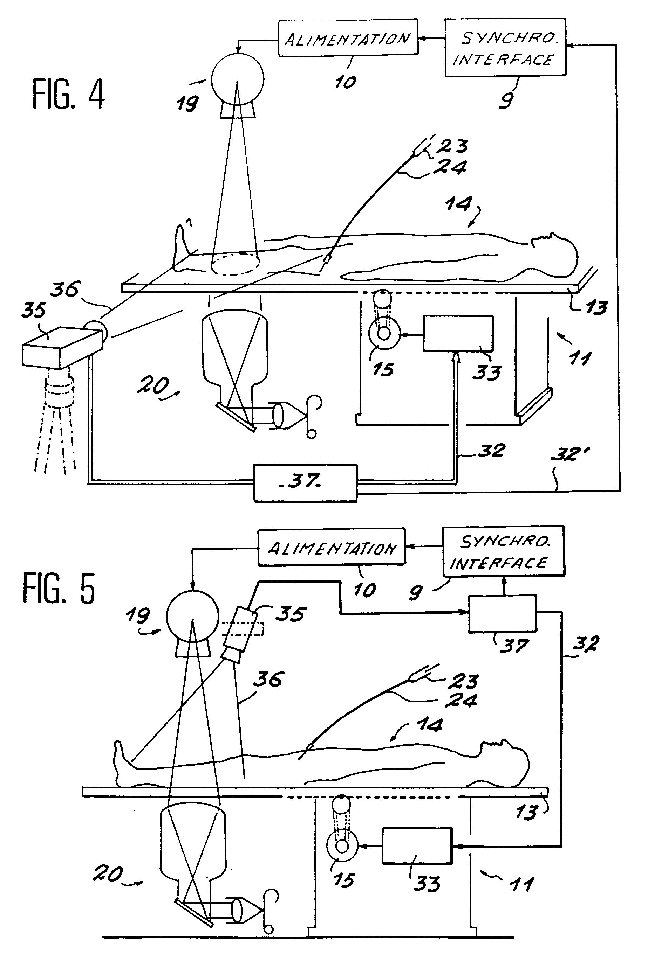

Cette vaso-dilatation a notamment pour effet d'augmenter la circulation sanguine périphérique par dilatation des petits vaisseaux et il en résulte une augmentation de la température de la peau du patient. L'invention propose de détecter cette élévation de température cutanée par des capteurs disposés sur au moins l'un des membres inférieurs, c'est le dispositif qui sera décrit en relation avec les figures 2 et 3. L'invention propose également de détecter cette élévation de température à l'aide d'une caméra de téléthermographie disposé sur un côté latéral du patient (figure 4) ou au-dessus du patient en association avec la source 19 de rayonnement X (figure 5).

La vaso-dilatation provoque également une augmentation du volume sanguin des couches périphériques des tissus et donc leur contenu en eau. L'invention propose alors d'utiliser des détecteurs qui sont sensibles au contenu en eau. Il en est ainsi des appareils à résonance magnétique nucléaire qui permettent de mesurer la densité de protons et donc d'eau. C'est le cas aussi des appareils qui mesurent les variations de résistivité en courant continu ou alternatif basse fréquence. Enfin, il existe des appareils qui mesurent les variations d'impédance pour des champs électriques modulés à très haute fréquence.

D'une manière générale, tout détecteur qui est sensible au passage du produit de contraste par ses effets induits est utilisable dans le cadre de l'invention.

Pour suivre la progression du produit de contraste, l'invention propose également de le rendre radio-actif et de détecter l'émission des rayons gamma par un appareil de scintigraphie par exemple.

Deux modes de réalisation d'un détecteur de passage d'embole seront maintenant décrits plus en détail en relation avec les figures 3 à 6, les signaux fournis par ces détecteurs étant utilisés pour déterminer d'abord la position de l'embole et pour changer ensuite, dans un système de radiodiagnostic pour examen angiographique, la position relative du patient et de l'ensemble source-récepteur.

Dans les différentes figures 1 à 5, les éléments identiques portent les mêmes références afin de rendre la description plus claire.

Le dispositif de détection 30 du système de radiodiagnostic de la figure 2 comprend une série de n capteurs C₁ à Cₙ sensibles aux modifications physiques induites par les modifications physiologiques qui sont provoquées par la propagation du produit de contraste. Les capteurs C₁ à Cₙ sont disposés sur l'une des jambes ou les deux suivant que l'on cherche à radiographier l'une ou les deux jambes. Les capteurs C₁ à Cₙ fournissent respectivement des signaux électriques S₁ à Sₙ qui sont appliqués à un dispositif de traitement 31 par l'intermédiaire de conducteurs F₁ à Fₙ. Le dispositif de traitement 31 fournit sur un conducteur 32 des signaux qui commandent, par l'intermédiaire d'un circuit de commande 33 du moteur 15, le déplacement du panneau 13. Par ailleurs, le dispositif 31 fournit sur un conducteur 32' des signaux qui commandent, par l'intermédiaire du dispositif 9 de synchronisation et d'interface et du dispositif 10 d'alimentation, la source 19 pour émettre des rayons X à des instants déterminés de manière à obtenir une image radiologique.

Les figures 3-a à 3-e sont les diagrammes des amplitudes des signaux S₁, S₂, S₃, S₄ et Sₙ en fonction du temps t, l'origine correspondant à l'instant d'injection du produit de contraste. Les signaux S₁ à Sₙ sont similaires entre eux mais sont décalés dans le temps, c'est-à-dire retardés l'un par rapport à l'autre, du fait qu'ils correspondent à des capteurs situés à des distances d₁, d₂, d₃, d₄...dₙ de plus en plus éloignées du point d'injection du produit de contraste. La forme de chaque signal est tel qu'il apparaît à un instant t₁, t₂, t₃, t₄ ou tₙ, augmente relativement rapidement jusqu'à une valeur maximale aux instants m₁, m₂, m₃, m₄...mₙ et décroît ensuite relativement lentement. Par suite de la dilution du produit de contraste au fur et à mesure de son déplacement, ses effets physiologiques sont de moins en moins accentués de sorte que les signaux S₁ à Sₙ sont de plus en plus larges, c'est-à-dire que les pentes de part et d'autre de la valeur maximale sont de plus en plus faibles lorsque l'indice n augmente.

Comme indiqué ci-dessus, le traitement des signaux S₁ à Sₙ est effectué par le dispositif 31 de manière à déterminer la position réelle de l'embole et l'instant d'avance du panneau 13 pour que chaque cliché comporte une image de l'embole. A cet effet, le programme de traitement tiendra compte du retard entre l'instant de passage de l'embole à la hauteur d'un capteur C2 par exemple, et celui d'apparition t₂ du début du signal S2 ou m₂ de sa valeur maximale. Pour réaliser ce programme, un modèle de propagation de l'embole sera établi par une étude statistique sur un grand nombre de cas.

Les capteurs qui peuvent être utilisés sont de plusieurs types selon les effets physiologiques qu'ils sont amenés à détecter.

Ainsi, si on met à profit l'élévation de la température cutanée, les capteurs sont du type thermométrique tels que des thermocouples. C'est le mode de réalisation de la figure 2.

On peut également mettre à profit les variations de résistivité en courant continu ou alternatif basse fréquence, variations qui permettent, par ailleurs, de réaliser des images dites de tomographie électrique. Un tel procédé est par exemple décrit dans l'article intitulé "ELECTRICAL IMPEDANCE TOMOGRAPHY - APPLIED POTENTIAL TOMOGRAPHY", de la revue : CLINICAL PHYSICS AND PHYSIOLOGICAL MEASUREMENT - Vol. 8 and 9 - 1988, publiée par The Institute of Physical Sciences in Medicine. La disposition des capteurs sera optimisée, soit pour être indicative du contenu global en eau dans une section des jambes, soit pour être indicative du contenu en eau dans les couches périphériques des jambes. Ce procédé est particulièrement efficace dans la mesure où le paramètre mesuré est sensible à la fois à la température et à la teneur en eau des tissus.

A la place de capteurs placés directement sur les jambes du patient, l'invention propose d'utiliser un ou des capteurs disposés à distance du patient et qui sont sensibles soit à l'élévation de température cutanée, soit à l'augmentation du volume sanguin des couches périphériques des tissus et donc de leur contenu en eau.

Dans les capteurs de la première catégorie, c'est-à-dire sensibles à l'élévation de température, l'invention propose d'utiliser une caméra de thermographie 35 (figure 4) dont le faisceau 36 vise l'ensemble des membres inférieurs du patient ou une zone limitée qui est située en amont de la zone dont on réalise une image radiologique. Les signaux électriques sous forme d'images fournis par la caméra 35 sont traités dans un dispositif de traitement 37 qui fournit alors, sur les conducteurs 32, des signaux d'avance du moteur 15 par l'intermédiaire du dispositif de commande 33 identique à celui de la figure 2 et, sur le conducteur 32' des signaux de commande de la source 19 par l'intermédiaire des dispositifs d'alimentation 10 et de synchronisation et d'interface 9.

Dans le mode de réalisation de la figure 4, la caméra 35 est disposée sur un support qui est placé d'un côté ou de l'autre de la table 11, ce qui la rend encombrante et gêne l'accès au patient. Aussi, l'invention propose de fixer la caméra 35 sur le tube 19 à rayons X de manière que son champ 36 contienne la totalité du champ radiographié augmenté au moins d'une zone équivalente audit champ radiographié en direction des pieds du patient.

On peut également utiliser des capteurs autres que ceux décrits ci-dessus en relation avec les figures 2 à 5.

On peut ainsi utiliser un appareil à résonance magnétique nucléaire qui permet de mesurer directement la densité de protons et donc d'eau. Dans un telle application, l'appareil à résonance magnétique nucléaire peut être réalisé de manière rudimentaire car il n'est pas nécessaire d'obtenir une image de haute qualité. Un tel appareil peut mettre en oeuvre des champs magnétiques de faible intensité qui sont réalisables par des aimants résistifs ou permanents.

On peut aussi utiliser des appareils qui détectent les variations d'impédance pour des champs électriques modulés à très haute fréquence dans la gamme des micro-ondes. Un tel appareil est par exemple décrit dans la thèse de Hugues de Talhouet soutenue le 19 décembre 1986 à l'Université de Paris-Sud et intitulée "CONTRIBUTION A L'AMELIORATION DE LA RESOLUTION EN IMAGERIE MICROONDE MONOCHROMATIQUE".

Les capteurs qui ont été décrits ci-dessus ont pour but de détecter les effets physiologiques induits par le passage du produit de contraste tels que l'élévation de température des tissus cutanés ou l'augmentation du contenu en eau des couches périphériques des tissus.

Des capteurs qui ne sont pas basés sur la détection d'effets physiologiques peuvent aussi être utilisés, par exemple un appareil de radiologie du type à rayons X qui détecterait le produit de contraste par son opacité plus importante. On peut aussi utiliser une variété spécifique de produit de contraste modifié pour émettre des rayons gamma et détecter son passage par un appareil de scintigraphie connu sous le nom de gamma-caméra.

## Revendications

1. Système de radiodiagnostic pour examen angiographique d'un patient (14) qui comprend un statif supportant une source (19) de rayonnement X et un récepteur (20) d'images radiologiques coopérant avec la source (19), une table de radiologie (11) comportant un panneau (13) monté sur un socle (12), des moyens (15 à 18) pour obtenir des déplacements relatifs du statif et du panneau 13, un dispositif (23,24) d'injection d'un produit de contraste dans le réseau sanguin du patient (14) et un dispositif d'acquisition des images détectées par le récepteur (20) de manière à obtenir finalement une image radiologique du corps du patient (14), caractérisé en ce qu'il comprend en outre, des moyens (C₁ à Cₙ,35) pour détecter la progression du produit de contraste dans le réseau sanguin du patient et des moyens (31 ou 37,33,9) pour déclencher, d'une part, le déplacement relatif du statif (19,20) et du panneau (13) en plusieurs positions et, d'autre part, la prise de l'image radiologique pour ces positions relatives de manière que l'image radiologique obtenue en chacune de ces positions relatives soit enregistrée à un instant où les vaisseaux sanguins de la zone dont on enregistre une image présentent une concentration significative du produit de contraste.

2. Système de radiodiagnostic selon la revendication 1, caractérisé en ce que les moyens (C₁ à Cₙ,35) pour détecter la progression du produit de contraste comprennent des capteurs qui sont sensibles aux effets physiologiques induits par le produit de contraste.

3. Système de radiodiagnostic selon la revendication 2, caractérisé en ce que les capteurs sont du type thermométrique de manière à détecter l'élévation de température des tissus cutanés.

4. Système de radiodiagnostic selon la revendication 3, caractérisé en ce que les capteurs sont des thermocouples (C₁ à Cₙ) qui sont disposés sur le corps du patient.

5. Système de radiodiagnostic selon la revendication 3, caractérisé en ce que les capteurs sont constitués par une caméra thermographique (35) disposée à distance du patient.

6. Système de radiodiagnostic selon la revendication 5, caractérisé en ce que la caméra thermographique (35) est disposée sur l'un ou l'autre côté longitudinal de la table de radiologie (11).

7. Système de radiodiagnostic selon la revendication 5, caractérisé en ce que la caméra tomographique (35) est disposée au-dessus de la table de radiologie (11) et est associée au tube (19) à rayonnement X.

8. Système de radiodiagnostic selon la revendication 2, caractérisé en ce que les capteurs sont du type à détecter les variations de résistivité en courant continu ou alternatif basse fréquence.

9. Système de radiodiagnostic selon la revendication 2, caractérisé en ce que les capteurs sont du type à détecter les variations d'impédance pour des champs électriques modulés à très haute fréquence.

10. Système de radiodiagnostic selon la revendication 2, caractérisé en ce que les capteurs sont du type à résonance magnétique nucléaire de manière à mesurer la variation de la densité d'eau.

11. Système de radiodiagnostic selon la revendication 4, caractérisé en ce que le produit de contraste comprend une substance radio-active et en ce que les capteurs sont du type à détecter le rayonnement gamma.

12. Système de radiodiagnostic selon la revendication 11, caractérisé en ce que les capteurs sont constitués par un appareil de scintigraphie.

13. Système de radiodiagnostic selon la revendication 1, caractérisé en ce que les moyens pour détecter la progression du produit de contraste sont constitués par un appareil de radiologie à rayonnement X qui détecte le produit de contraste par son opacité au rayonnement X.

## Claims

1. An x-ray diagnostics system for the angiographic examination of a patient (14) comprising a stand bearing an x-ray source (19) and a receiver (20) for x-ray images cooperating with the source (19), an x-ray examination table (11) having stage (13) mounted on a base (12), means (15 through 18) to produce relative displacements of the stand and of the stage 13, a device (23, 24) for the injection of a contrast agent into the patient's blood circulation (14) and a device for capturing images detected by the receiver (20) in such a manner as to finally obtain an x-ray image of the body of the patient (14), characterized in that it furthermore comprises means (C1 through Cn, 35) to detect the propagation of the contrast agent in the blood circulation system of the patient and means (31 or 37, 33, 9) in order to cause, on the one hand the relative displacement of the stand (19, 20) and of the stage (13) into a plurality of positions and, on the other hand, the production of an x-ray image for such relative positions in such a manner that the x-ray image produced in every one of such relative positions is recorded at an instant at which the blood vessels of the zone, whose image is to be produced, have a significant concentration of the contrast agent.

2. The x-ray diagnostics system as claimed in claim 1, characterized in that the means (C1 through Cn, 35) for detecting the propagation of the contrast agent comprise sensors which are sensitive to the physiological effects induced by the contrast agent.

3. The x-ray diagnostics system as claimed in claim 2, characterized in that the sensors are of the thermometric type in such a manner as to detect the elevation of the temperature of cutaneous tissues.

4. The x-ray diagnostics system as claimed in claim 3, characterized in that the sensors are thermcouples (C1 through Cn) which are arranged on the body of the patient.

5. The x-ray diagnostics system as claimed in claim 3, characterized in that the sensors are constituted by a thermographic camera (34) placed at a distance from the patient.

6. The x-ray diagnostics system as claimed in claim 5, characterized in that the thermographic camera (35) is placed on one or the other longitudinal side of the x-ray examination table (11).

7. The x-ray diagnostics system as claimed in claim 5, characterized in that the tomographic camera (35) is arranged above the x-ray examination table (11) and is associated with the x-ray tube (19).

8. The x-ray diagnostics system as claimed in claim 2, characterized in that the sensors are of the DC or low frequency AC variable resistivity type.

9. The x-ray diagnostics system as claimed in claim 2, characterized in that the sensors are of the type adapted to detect variations in impedance using modulated VHF electrical fields.

10. The x-ray diagnostics system as claimed in claim 2, characterized in that the sensor are of the nuclear magnetic resonance type such as to measure variations in the density of water.

11. The x-ray diagnostics system as claimed in claim 4, characterized in that the contrast agent comprises a radioactive substance and in that the sensors are of the type adapted to detect gamma radiation.

12. The x-ray diagnostics system as claimed in claim 11, characterized in that the sensors are constituted by a scintigraphic system.

13. The x-ray diagnostics system as claimed in claim 1, characterized in that the means for the detection of the propagation of the contrast agent are constituted by an x-ray apparatus which detects the contrast agent owing to its opacity to x-rays.

## Patentansprüche

1. Röntgenstrahldiagnostisches Verfahren zur angiographischen Untersuchung eines Patienten (14), mit einem Stativ, das eine Röntgenstrahlquelle (19) und einen Empfänger (20) für Röntgenbilder hält, der mit der Quelle (19) zusammenwirkt, einem radiologischen Tisch (11) mit einer auf einem Sockel (12) montierten Platte (13), Mitteln (15 bis 18) zur Erzielung von Relativverschiebungen des Stativs und der Platte (13), einer Einrichtung (23, 24) zum Einspritzen eines Kontrastmittels in den Blutkreislauf des Patienten (14) sowie einer Einrichtung zum Erfassen der von dem Empfänger (20) detektierten Bilder derart, daß schließlich ein Röntgenbild des Körpers des Patienten (14) erhalten wird, dadurch gekennzeichnet, daß es ferner Mittel (C₁ bis Cₙ, 35) aufweist, um das Fortschreiten des Kontrastmittels in dem Blutkreislauf des Patienten zu detektieren, sowie Mittel (31 oder 37, 33, 9), um einerseits die Relativerschiebung des Stativs (19, 20) und der Platte (13) in mehreren Positionen und andererseits die Aufnahme des Röntgenbildes für diese Relativpositionen derart auszulösen, daß das an jeder dieser Relativpositionen erhaltene Röntgenbild zu einem Zeitpunkt aufgezeichnet wird, wo die Blutgefäße der Zone, deren Bild aufgezeichnet wird, eine deutliche Konzentration des Kontrastmittels zeigen.

2. Röntgenstrahldiagnostisches Verfahren nach Anspruch 1, dadurch gekennzeichnet, daß die Mittel (C₁ bis Cₙ, 35) zum Detektieren des Fortschreitens des Kontrastmittels Sensoren aufweisen, die gegenüber den durch das Kontrastmittel induzierten physiologischen Effekten empfindlich sind.

3. Röntgenstrahldiagnostisches Verfahren nach Anspruch 2, dadurch gekennzeichnet, daß die Sensoren vom thermometrischen Typ sind, so daß sie die Temperaturerhöhung der Hautgewebe detektieren.

4. Röntgenstrahldiagnostisches Verfahren nach Anspruch 3, dadurch gekennzeichnet, daß die Sensoren Thermoelemente (C₁ bis Cₙ) sind, die am Körper des Patienten angeordnet sind.

5. Röntgenstrahldiagnostisches Verfahren nach Anspruch 3, dadurch gekennzeichnet, daß die Sensoren durch eine Thermographiekamera (35) gebildet sind, die im Abstand zum Patienten angeordnet ist.

6. Röntgenstrahldiagnostisches Verfahren nach Anspruch 5, dadurch gekennzeichnet, daß die Thermographiekamera (35) an der einen oder anderen Längsseite des Röntgentisches (11) angeordnet ist.

7. Röntgenstrahldiagnostisches Verfahren nach Anspruch 5, dadurch gekennzeichnet, daß die Tomographiekamera (35) über dem Röntgentisch (11) angeordnet und der Röntgenstrahlröhre (19) zugeordnet ist.

8. Röntgenstrahldiagnostisches Verfahren nach Anspruch 2, dadurch gekennzeichnet, daß die Sensoren von dem Typ zum Detektieren der Resistivitätsschwankungen bei niederfrequentem Gleich- oder Wechselstrom sind.

9. Röntgenstrahldiagnostisches Verfahren nach Anspruch 2, dadurch gekennzeichnet, daß die Sensoren von dem Typ zum Detektieren der Impedanzschwankungen für mit Höchstfrequenz modulierte elektrische Felder sind.

10. Röntgenstrahldiagnostisches Verfahren nach Anspruch 2, dadurch gekennzeichnet, daß die Sensoren vom Kernspinresonanztyp sind, um die Schwankung der Wasserdichte zu messen.

11. Röntgenstrahldiagnostisches Verfahren nach Anspruch 4, dadurch gekennzeichnet, daß das Kontrastmittel eine radioaktive Substanz enthält und die Sensoren von dem Typ zum Detektieren der Gammastrahlung sind.

12. Röntgenstrahldiagnostisches Verfahren nach Anspruch 11, dadurch gekennzeichnet, daß die Sensoren durch eine Szintigraphievorrichtung gebildet sind.

13. Röntgenstrahldiagnostisches Verfahren nach Anspruch 1, dadurch gekennzeichnet, daß die Mittel zum Detektieren des Fortschreitens des Kontrastmittels durch eine radiologische Vorrichtung mit Röntgenstrahlung gebildet sind, die das Kontrastmittel durch seine Undurchlässigkeit gegenüber der Röntgenstrahlung detektiert.
